(19) Europäisches Patentamt European Patent Office Office européen des brevets

(11) **EP 3 881 898 A1**

(12) **EUROPEAN PATENT APPLICATION**
published in accordance with Art. 153(4) EPC

(43) Date of publication:
**22.09.2021 Bulletin 2021/38**

(21) Application number: **19883964.9**

(22) Date of filing: **12.11.2019**

(51) Int Cl.:
*A61P 25/16* (2006.01)    *A61P 25/28* (2006.01)
*A61K 36/42* (2006.01)    *A23L 33/105* (2016.01)
*A23L 33/11* (2016.01)    *A61K 8/9789* (2017.01)

(86) International application number:
**PCT/JP2019/044414**

(87) International publication number:
**WO 2020/100917 (22.05.2020 Gazette 2020/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.11.2018 JP 2018213172**

(71) Applicants:
• **Green Tech Co., Ltd.**
**Kyoto-shi, Kyoto 602-0915 (JP)**
• **MCB., Co.**
**Hirado-shi, Nagasaki 859-5114 (JP)**

(72) Inventors:
• **SUGIMOTO, Hachiro**
**Kyoto-shi Kyoto 602-0915 (JP)**
• **NAGASHIMA, Koki**
**Hirado-shi, Nagasaki 859-5121 (JP)**
• **OKUDA, Michiaki**
**Kyoto-shi Kyoto 602-0915 (JP)**
• **FUJITA, Yuki**
**Kyoto-shi Kyoto 602-0915 (JP)**

(74) Representative: **Lederer & Keller Patentanwälte Partnerschaft mbB**
**Unsöldstraße 2**
**80538 München (DE)**

(54) **PROPHYLACTIC AGENT AND/OR THERAPEUTIC AGENT FOR NEURODEGENERATIVE DISEASE**

(57)    The problems to be solved by the present invention are to provide a novel agent for preventing and/or treating a neurodegenerative disease, and a pharmaceutical composition comprising the agent for preventing and/or treating a neurodegenerative disease. The problems are solved by providing an agent for preventing and/or treating a neurodegenerative disease, the agent comprising as an active ingredient an extract of *Momordica charantia* var. *pavel,* and preferably the agent is for use in treatment or prevention of a disease selected from Alzheimer's disease, Parkinson's disease, Down syndrome, tauopathy (for example, argyrophilic grain dementia, senile dementia of neurofibrillary tangle type, progressive supranuclear palsy, degeneration of cerebral cortex or Pick disease), and dementia with lewy bodies.

Fig. 2

**Description**

TECHNICAL FIELD

The present invention relates to an agent for preventing and/or treating a neurodegenerative disease.

BACKGROUND ART

Neurodegenerative diseases are caused by gradual impairment of specific nerve cell groups (including, for example, nerve cells involved in cognitive function and cells involved in motor function) among nerve cells in the brain or spinal cord. Many studies have been conducted to develop curative or ameliorative therapy for these diseases.
For example, Patent literature 1 reports that a combination of a glycerophospholipid and ferulic acid, which is found in coffee, onion, Japanese radish, lemon, *Cnidium* rhizome, Japanese angelica root, pine, *Coptis* rhizome, *Ferula assa-foetida, Nardostachys* rhizome, corn, barley, wheat, rice, etc., exhibits inhibitory effect on amyloid-β aggregation.
In addition to the plant-derived component as described above, an extract from the seeds of *Nerium* species or *Thevetia* species can also be used for treatment of Alzheimer's disease, Huntington's disease, cerebral apoplexy or other neurological diseases (Patent literature 2).
Bitter melon *(Momordica charantia* var. *pavel;* hereinafter called *Momordica charantia)* is a plant of the family *Cucurbitaceae* grown in tropical Asia, and is widely cultivated in the Okinawa and Kyushu regions of Japan. *Momordica charantia* is rich in nutrients such as amino acids, vitamins and minerals, and is expected to be effective for prevention of summer fatigue, promotion of appetite, assistance of digestion, regulation of intestines, and enhancement of cell activation. Due to these potential benefits, *Momordica charantia* has been studied for its application to processed foods (for example, see Patent literatures 3 and 4).

CITATION LIST

PATENT LITERATURE

Patent literature 1: JP 2017-186335 A
Patent literature 2: JP 2017-114888 A
Patent literature 3: JP H6-141813 A
Patent literature 4: WO 2017/022030

SUMMARY OF INVENTION

TECHNICAL PROBLEM

Despite of many reports, an extract of *Momordica charantia* is not known to have the effect of preventing and/or curing neurodegenerative diseases.
The present invention relates to a novel agent for preventing and/or treating a neurodegenerative disease, and a pharmaceutical composition comprising the agent for preventing and/or treating a neurodegenerative disease. The agent is administered to an animal subject, including humans, pets such as dogs and cats, and livestock animals such as cows and pigs.

SOLUTION TO PROBLEM

The inventors conducted extensive studies to solve the above problems, and as a result found that an extract of *Momordica charantia* has the effect of inhibiting aggregation of tau protein and amyloid-β protein, which are causative factors of neurodegenerative diseases including Alzheimer's disease, and the inventors completed the invention.
That is, the present invention relates to the following (1) to (7).

(1) An agent for preventing and/or treating a neurodegenerative disease, the agent comprising as an active ingredient an extract of *Momordica charantia* var. *pavel.*
(2) The agent according to the above (1) for use in treatment or prevention of a disease selected from Alzheimer's disease, Parkinson's disease, Down syndrome, tauopathy, and dementia with lewy bodies.
(3) A composition comprising the agent according to the above (1) or (2).

(4) The composition according to the above (3), which is a pharmaceutical composition.

(5) The composition according to the above (3), which is a food or drink composition.

(6) The composition according to the above (3), which is a cosmetic composition.

(7) The composition according to any one of the above (3) to (6) for use in prevention and/or treatment of Alzheimer's disease.

ADVANTAGEOUS EFFECTS OF INVENTION

The agent for preventing and/or treating a neurodegenerative disease according to the present invention exhibits the excellent effect of preventing the onset and/or progression of cognitive dysfunctions via its inhibitory activity on protein aggregation and other activities.

BRIEF DESCRIPTION OF DRAWINGS

Fig. 1 is a chart showing the inhibition of tau protein aggregation.

Fig. 2 is a chart showing the inhibition of amyloid-β protein aggregation.

DESCRIPTION OF EMBODIMENTS

The agent for preventing and/or treating a neurodegenerative disease according to the present invention comprises as an active ingredient an extract of *Momordica charantia.* Hereinafter, the extract of *Momordica charantia* may also be called a *Momordica charantia* extract or an extract of the present invention. The agent for preventing and/or treating a neurodegenerative disease may also be simply called the preventive and/or therapeutic agent of the present invention.

The neurodegenerative disease as described herein may be any disease in which damage occurs due to protein aggregation in nerve cells, but is not limited thereto. The present invention will be described in an exemplary embodiment wherein the neurodegenerative disease is Alzheimer's disease.

Alzheimer's disease is a neurodegenerative disease characterized by hallmark lesions composed of senile plaques of amyloid-β protein and neurofibrillary tangles of tau protein, as well as progressive brain atrophy associated with these lesions. Amyloid-β protein forms aggregates and deposits in tissues to develop functional disorders. Phosphorylated tau protein also aggregates and deposits in tissues to develop functional disorders. The extract of the present invention has inhibitory effect on aggregation of these proteins, and is thus effective for inhibition of the onset and progression of Alzheimer's disease and is also potentially effective for neurodegenerative diseases, such as diseases in which damage occurs due to protein aggregation in nerve cells.

The extract of the present invention is prepared from *Momordica charantia,* but any materials prepared from the plant by an artificial treatment can be used in the present invention. Examples of materials prepared from the plant include, in addition to an extract, an essential oil (for example, an essential oil prepared by steam distillation, a squeezed juice, a pressed juice, an extract prepared by solvent extraction, or an extract prepared by supercritical fluid extraction) . These materials can be used alone or in combination of two or more types.

The *Momordica charantia* used in the present invention may be from any origin, including, but not limited to, Europe, North Africa, Asia, North and South America, etc. For example, the *Momordica charantia* may be from Japan, in particular, from Hirado city.

The part used of the *Momordica charantia* may be, for example, rhizomes, leaves, fruits, seeds, flowers, or the whole plant. Processed products of these parts, such as cut pieces or a pulverized product or a dried product thereof, can also be used.

The extract used in the present invention can be prepared by subjecting *Momordica charantia* as described above to extraction process with a solvent in accordance with a known method, and then evaporating off the solvent.

Examples of the extraction solvent include lower alcohols (methanol, ethanol, propanol, isopropyl alcohol, n-butanol, isobutyl alcohol, etc.), polyalcohols (ethylene glycol, propylene glycol, 1, 3-butylene glycol, etc.), ketones (acetone, methyl ethyl ketone, etc.), ethers (dioxane, methyl ethyl ether, diethyl ether, etc.), halogenated hydrocarbons (chloroform, dichloromethane, dichloroethane, etc.), polar solvents (dimethylformamide, dimethylsulfoxide, etc.), as well as various organic solvents, such as ethyl acetate, toluene, n-hexane, and petroleum ether, and water. These solvents can be used alone or in combination of two or more types.

The extraction temperature may vary with the solvent used, the part or form of the plant used and other factors, and cannot be finitely defined. However, for efficient extraction, the lower end of the range of extraction temperature may be 4°C, and the higher end of the range of extraction temperature may be 150°C. The lower and higher ends of the range of extraction temperature in the present invention may be appropriately selected within the above temperature range. For example, the lower and higher ends of the range of extraction temperature may be selected from 10°C, 15°C, 20°C, 30°C, 40°C, 60°C, 80°C, 100°C, 120°C, 140°C, etc. The extraction time can be selected as appropriate depending on the amount of the material subjected to the extraction and the device used for the extraction.

The extract obtained by the extraction can further be subjected to one or more treatments selected from the group consisting of filtration, centrifugation, concentration, ultrafiltration, lyophilization, pulverization and fractionation, in accordance with a known method. These treatments can be performed under normal pressure or reduced pressure, if necessary. An aspect of the extract of the present invention includes a product obtained by said one or more treatments. One should be noted that *Momordica charantia* is known to contain quinine, but quinine has adverse side effects that cause optic nerve damage, hematological disorders, etc., and therefore the extract of the present invention is preferably substantially free of quinine. Accordingly, quinine may be removed by a known method during preparation of the extract of the present invention, or alternatively, the extract of the present invention may be prepared using, as a raw material, a plant variety that does not contain quinine, or alternatively, the extract of the present invention may be prepared using, as a raw material, a plant variety that does not contain quinine and then removal of quinine is performed by a known method. The term "substantially free of" as used herein means that the amount of the substance in the extract should be 0.5% by weight or less, preferably 0.1% by weight or less.

The extract prepared as described above preferably contains momordicin, charantin, vitamin C, β-carotene, potassium, magnesium, iron, sodium, calcium, etc. In addition to these, the extract may also contain, for example, alkaloids, charin, cryptoxanthin, cucurbitin, cucurbitacin, cucurbitane, cycloartenol, diosgenin, eleostearic acid, erythrodiol, galacturonic acid, gentisic acid, goyaglycosides, goyasaponin, guanylate cyclase inhibitors, gypsogenin, hydroxytryptamine, karounidiol, lanosterol, lauric acid, linoleic acid, linolenic acid, momorcharaside, momorcharin, momordenol, momordicin, momordicinin, momordicoside, momordin, multiflorenol, myristic acid, nerolidol, oleanolic acid, oleic acid, oxolinic acid, pentadecanes, peptides, petroselinic acid, polypeptides, proteins, ribosome-inactivating proteins, rosmarinic acid, rubixanthin, spinasterol, steroidal glycosides, stigmastadiol, stigmasterol, taraxerol, trehalose, trypsin inhibitors, uracil, vatine, insulin, verbascoside, bithin, zeatin, zeatin riboside, zeaxanthin, zeinoxanthin, etc. The amounts of these components in the extract are not limited to particular ones, and may be adjusted by a combination of various treatments, including partition extraction, concentration, and chromatographic purification.

The extract of the present invention may be in any form, such as a powder, a solid or a liquid. The extract of the present invention may also be in the form of solid granules produced by a known granulation method. Examples of the granulation method include, but are not limited to, tumbling granulation, agitation granulation, fluidized bed granulation, steam-jet granulation, extrusion granulation, compression granulation, disintegrating granulation, prilling granulation and spray granulation. The extract in a liquid form may be, for example, a liquid extract obtained by the preparation method as described above, a concentrate or a dilution thereof, or a liquid prepared by dissolving a powdered extract in a liquid, such as water and an alcohol.

The preventive and/or therapeutic agent of the present invention may be any type of agent that comprises as an active ingredient an extract of *Momordica charantia*. The amount of an extract of *Momordica charantia* contained in the agent is, for example, 0.1 to 100% by weight.

The preventive and/or therapeutic agent of the present invention is capable of inhibiting aggregation of aggregate-prone proteins, such as amyloid-β protein and tau protein, in nerve cells. Examples of the aggregate-prone proteins include amyloid-β protein and tau protein, as well as α-synuclein. The preventive and/or therapeutic agent of the present invention can therefore be used to prevent, improve or treat, for example, Alzheimer's disease, Parkinson's disease, Down syndrome, tauopathy (for example, argyrophilic grain dementia, senile dementia of neurofibrillary tangle type, progressive supranuclear palsy, degeneration of cerebral cortex or Pick disease), dementia with lewy bodies, etc. The preventive and/or therapeutic agent of the present invention can also be suitable for other diseases whose secondary symptoms include the symptoms of the above diseases. Specific examples of said other diseases include, for example, systemic lupus erythematosus (SLE).

The preventive and/or therapeutic agent of the present invention may be in any form that allows the extract of the present invention to act on nerve cells in the brain. The agent may be directly formulated into a dosage form, or prepared into a form that can be used as an ingredient for pharmaceuticals, quasi-drugs, foods or drinks, food or drink additives, cosmetics, etc.

The present invention also provides a composition comprising the preventive and/or therapeutic agent of the present invention. The composition may be in any form that allows the extract of the present invention to be taken by nerve cells. The composition is suitable as, for example, a pharmaceutical composition, a food or drink composition, or a cosmetic composition.

The pharmaceutical composition can be utilized as various types of pharmaceuticals, quasi-drugs, etc. Specific examples of the pharmaceuticals include oral and parenteral formulations, such as tablets (including uncoated tablets, sugar-coated tablets, film-coated tablets, sublingual tablets, orally disintegrating tablets, buccal tablets, etc.), pills, powders, granules, capsules (including soft capsules and microcapsules), troches, syrups, liquids, emulsions, suspensions, controlled release formulations (for example, fast release formulations, sustained release formulations, and sustained release microcapsules), aerosols, films (for example, orally disintegrating films, and oral mucosal adhesive films), transdermal formulations, ointments, lotions, patches, pellets, transnasal formulations, transpulmonary formulations (inhalants), etc. The pharmaceutical composition of the present invention can be prepared according to the conventional method by appropriately combining the preventive and/or therapeutic agent of the present invention with a carrier, a base and/or an additive, etc. commonly used in the pharmaceutical field in an amount that allows the objects of the present invention to be achieved.

The food or drink composition can be utilized as various types of foods or drinks, or food or drink additives. Specific examples of the foods or drinks include various types of foods such as bakery products, cakes, noodles, sweets, jellies, frozen foods, ice creams, dairy products, drinks, soups, and edible oils; as well as fruit juices, carbonated drinks, tea drinks, sports drinks, milk drinks, alcoholic drinks, and refreshing drinks. The foods or drinks also include foods with function claims, foods with nutrient function claims, foods for special health use, foods for special dietary use, foods for elderly people, foods for sick people, and dietary supplements (supplements) . These foods and drinks may be in any form, and may be in oral dosage forms as exemplified above for the pharmaceuticals and the quasi-drugs. The foods and drinks may be produced by adding the preventive and/or therapeutic agent of the present invention to an existing food or drink product during or after the preparation of the preventive and/or therapeutic agent. The timing or manner of addition of the agent to an existing food or drink product is not limited to a particular one.

Specific examples of the cosmetic composition include washing lotions, shampoos, rinses, hair tonics, hair lotions, aftershave lotions, body lotions, makeup lotions, cleansing creams, massage creams, emollient creams, aerosol products, fragrances, and bath fragrances. The cosmetic composition of the present invention can be prepared according to the conventional method by appropriately combining the preventive and/or therapeutic agent of the present invention with a carrier, a base and/or an additive, etc. commonly used in the cosmetic field in an amount that allows the objects of the present invention to be achieved.

Since the composition of the present invention comprises the preventive and/or therapeutic agent of the present invention, the composition is suitable for diseases for which the preventive and/or therapeutic agent of the present invention is effective. For example, the composition of the present invention can be suitable for preventing, improving or treating Alzheimer's disease, Parkinson's disease, Down syndrome, tauopathy (for example, argyrophilic grain dementia, senile dementia of neurofibrillary tangle type, progressive supranuclear palsy, degeneration of cerebral cortex or Pick disease), dementia with lewy bodies, etc. For example, the composition of the present invention can be provided as a food or drink composition for preventing or improving a neurodegenerative disease, the composition comprising as an active ingredient an extract of *Momordica charantia.*

The amount of an extract of *Momordica charantia* contained in the composition of the present invention varies depending on the form of the composition, the mode of ingestion, the carrier used, etc., but is, for example, typically 0.01 to 100% by weight, preferably 0.05 to 90% by weight, more preferably 0.1 to 50% by weight, and further preferably 0.1 to 30% by weight based on the total weight of the composition.

The dosage of the composition ingested or consumed varies depending on the subject, the symptoms, the mode of ingestion or consumption, etc., but the dosage for oral administration to, for example, a human with a body weight of about 60 kg is, for example, typically about 0.01 to 1000 mg per day, preferably about 0.1 to 100 mg per day, and more preferably about 0.5 to 50 mg per day, in terms of the dry weight of an extract of *Momordica charantia*. The total daily dosage may be administered in a single dose or several divided doses.

The composition of the present invention comprises *Momordica charantia,* which has long been consumed as a food, and is thus highly safe. Therefore the composition of the present invention may be used in combination with a known therapeutic medicine for a neurodegenerative disease, including, for example, Alzheimer's disease, Parkinson's disease, Down syndrome, tauopathy (for example, argyrophilic grain dementia, senile dementia of neurofibrillary tangle type, progressive supranuclear palsy, degeneration of cerebral cortex or Pick disease), dementia with lewy bodies, frontotemporal lobar degeneration, depression, bipolar disorder, anxiety disorder, panic disorder, schizophrenia, autism, traumatic nerve damage, ischemic encephalopathy, etc. The composition of the present invention, when used in combination with such a known therapeutic medicine for a neurodegenerative disease, is expected to exhibit enhanced additive or synergistic effect.

The present invention can also be embodied as a feed or a feed additive comprising the preventive and/or therapeutic agent of the present invention.

The present invention also provides a method for preventing and/or treating a neurodegenerative disease, the method comprising administering to a subject the preventive and/or therapeutic agent of the present invention. The neurodegenerative disease may be those exemplified as diseases for which the preventive and/or therapeutic agent of the present invention is suitable.

In an aspect of the present invention, since an extract of *Momordica charantia* is capable of inhibiting protein aggregation, the present invention also provides use of an extract of *Momordica charantia* for prevention and/or treatment of a neurodegenerative disease, and use of an extract of *Momordica charantia* for production of an agent for preventing and/or treating a neurodegenerative disease.

EXAMPLES

The present invention will be described in more detail below with reference to Examples, but the present invention is not limited thereto.

Example 1

The leaves of *Momordica charantia* (harvested in Hirado city) were subjected to extraction with ethanol. The extract was concentrated, and diluted in purified water so that the final concentration was 20-, 200- or 2000-fold the concentration of the stock solution (the *Momordica charantia* extract) to prepare test substances.

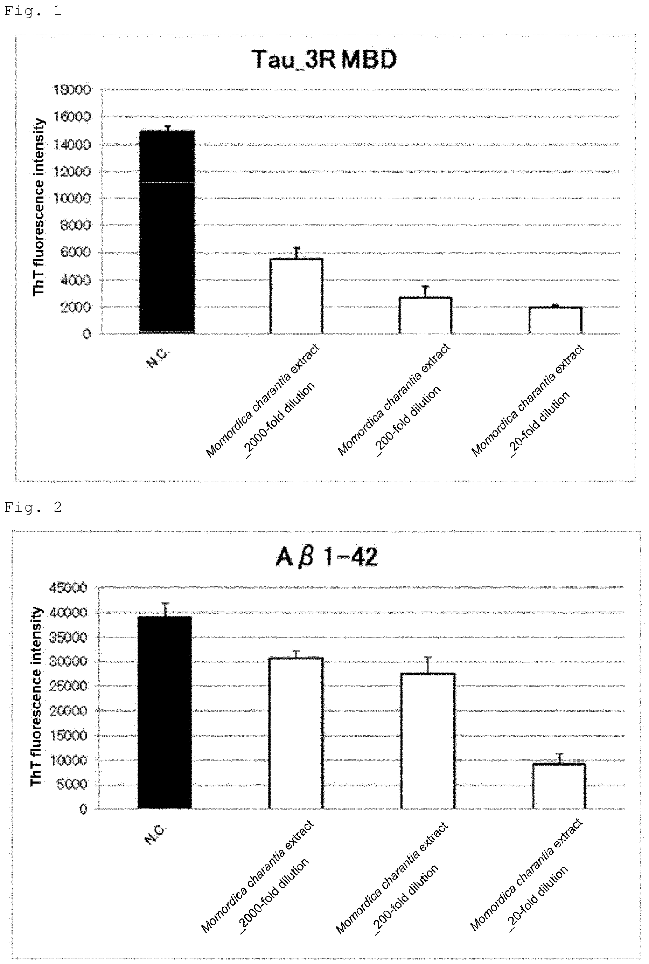

To 10 $\mu$L of each of the test substances were added 150 $\mu$L of 50 mM Tris-HCl (pH 7.6), 20 $\mu$L of 100 $\mu$M tau protein (3R MBD), and 20 $\mu$L of 100 $\mu$M heparin, and the mixture was incubated at 37°C for 16 hours under protection from light. After completion of incubation, 15 $\mu$L of 100 $\mu$M thioflavin T solution was added to 135 $\mu$L of the reaction mixture, and the fluorescence intensity was measured with a microplate reader (Fig. 1). The percent inhibition of tau protein aggregation was calculated from the measured values using the formula below. The results are shown in Table 1 below.

$$\text{Inhibition of aggregation (\%)} = 100 - 100 \times \{(\text{Measured value of test substance} - \text{autofluorescence})/(\text{measured value of negative control} - \text{autofluorescence})\}$$

Table 1

| *Momordica charantia* extract | Inhibition of aggregation (%) |
|---|---|
| 2000-fold dilution | 63.3 |
| 200-fold dilution | 81.6 |
| 20-fold dilution | 86.7 |

The results demonstrate that the *Momordica charantia* extract inhibited tau protein aggregation as compared to the negative control (N.C.). The effect was dose-dependent.

Example 2

Test substances were prepared in the same manner as in Example 1 except for using PBS for dilution.

Amyloid-$\beta$ protein (A$\beta$ 1-42) (Peptide Institute, Inc., 4349-v) was dissolved in 0.1% NH$_3$ water and diluted in PBS to a concentration of 20 $\mu$M to prepare a substrate solution. Then, 25 $\mu$L of the substrate solution was added to 25 $\mu$L of each of the test substances, and the mixture was incubated at 37°C for 24 hours under protection from light. After completion of incubation, 50 $\mu$L of 6 $\mu$M ThT solution was added to the reaction mixture, and the fluorescence intensity was measured with a microplate reader (Fig. 2). The percent inhibition of amyloid-$\beta$ protein aggregation was calculated from the measured values. The results are shown in Table 2 below.

Table 2

| *Momordica charantia* extract | Inhibition of aggregation (%) |
|---|---|
| 2000-fold dilution | 21.4 |
| 200-fold dilution | 29.5 |
| 20-fold dilution | 76.6 |

The results demonstrate that the *Momordica charantia* extract inhibited amyloid-$\beta$ protein aggregation as compared to the negative control (N.C.). The effect was dose-dependent.

Example 3

Two capsules each containing 2.5 mg of a *Momordica charantia* extract were administered every morning to a woman of 75 years old suffering from systemic lupus erythematosus (SLE), and improvement of the cognitive function was investigated. Specifically, it was examined to what extent the symptoms were improved as compared with those before administration in terms of the following five items: (1) telling the same things repeatedly, (2) wandering during the night, (3) struggling to follow a conversation, (4) difficulty concentrating, and (5) difficulty hearing.
The results demonstrate that the items (1) to (4) were improved over the duration of administration, in particular, the symptoms were reduced to about 40% one month later, about 60% two months later, and about 80 % three months later. The item (5) was also confirmed to be improved and was reduced to about 30 to 40% three months later.

INDUSTRIAL APPLICABILITY

The preventive and/or therapeutic agent of the present invention exhibits protective effect on nerve cells via inhibition of protein aggregation in nerve cells, and is therefore suitable as a preventive and/or therapeutic agent for neurodegenerative diseases, including, inter alia, Alzheimer's disease, Parkinson's disease, Down syndrome, tauopathy (for example, argyrophilic grain dementia, senile dementia of neurofibrillary tangle type, progressive supranuclear palsy, degeneration of cerebral cortex or Pick disease), and dementia with lewy bodies.

**Claims**

1. An agent for preventing and/or treating a neurodegenerative disease, the agent comprising as an active ingredient an extract of *Momordica charantia* var. *pavel.*

2. The agent according to claim 1 for use in treatment or prevention of a disease selected from Alzheimer's disease, Parkinson's disease, Down syndrome, tauopathy, and dementia with lewy bodies.

3. A composition comprising the agent according to claim 1 or 2.

4. The composition according to claim 3, which is a pharmaceutical composition.

5. The composition according to claim 3, which is a food or drink composition.

6. The composition according to claim 3, which is a cosmetic composition.

7. The composition according to any one of claims 3 to 6 for use in prevention and/or treatment of Alzheimer's disease.

Fig. 1

Fig. 2

## INTERNATIONAL SEARCH REPORT

| International application No. |
| --- |
| PCT/JP2019/044414 |

**A. CLASSIFICATION OF SUBJECT MATTER**

A61P 25/16(2006.01)i; A61P 25/28(2006.01)i; A61K 36/42(2006.01)i; A23L 33/105(2016.01)i; A23L 33/11(2016.01)i; A61K 8/9789(2017.01)i
FI: A61K36/42; A61P25/28; A61P25/16; A23L33/105; A23L33/11; A61K8/9789

**B. FIELDS SEARCHED**

Minimum documentation searched (classification system followed by classification symbols)
A61P25/16; A61P25/28; A61K36/42; A23L33/105; A23L33/11; A61K8/9789

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | KR 10-2015-0075047 A (HAMYANG AGRICULTUTAL UNION CORPORATION) 02.07.2015 (2015-07-02) claims, examples, test example 3 | 1-7 |
| X | CHO, E. J. et al., "Protective effect of protocatechuic acid of Momordica charantia from memory impairment induced by amyloid beta25-35", Faseb J, 01 April 2013, vol. 27, no. 1, supplement, p. 661.6, in particular, abstract | 1-7 |
| X | TAMILANBAN, T. et al., "Isolation, characterization, and memory enhancing activity of charantin using zebrafish model", J Pharm Res, 01 April 2018, vol. 12, issue 4, pp. 648-655, in particular, abstract | 1-7 |

☒ Further documents are listed in the continuation of Box C.     ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 16 January 2020 (16.01.2020) | 04 February 2020 (04.02.2020) |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

| International application No. |
| --- |
| PCT/JP2019/044414 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | CN 107556362 A (ZHEJIANG UNIVERSITY) 09.01.2018 (2018-01-09) claims, examples 1, 2 | 1-7 |
| X | CN 102526090 A (XUZHOU MEDICAL COLLEGE) 04.07.2012 (2012-07-04) claims, examples | 1-7 |
| X | 岡田悦政, ほか1名, 植物種子抽出によるアミロイドβの減少, 日本家政学会 第64回大会研究発表要旨集, vol. 64, 2012.05.01, p. 59 (OKADA, Yoshinori et al., "Decrease of amyloid-beta level by plant seeds extracts", Presentation abstracts of 64th Annual Congress of The Japan Society of Home Economics) | 1-7 |
| P,X | CN 108887448 A (GUANGZHOU GUOYU MEDICAL TECHNOLOGY CO., LTD.) 27.11.2018 (2018-11-27) claims, examples, experiment examples | 1,3-6 |

Form PCT/ISA/210 (continuation of second sheet) (January 2015)

## INTERNATIONAL SEARCH REPORT
Information on patent family members

| International application No. |
| --- |
| PCT/JP2019/044414 |

| Patent Documents referred in the Report | Publication Date | Patent Family | Publication Date |
| --- | --- | --- | --- |
| KR 10-2015-0075047 A | 02 July 2015 | (Family: none) | |
| CN 107556362 A | 09 Jan. 2018 | (Family: none) | |
| CN 102526090 A | 04 Jul. 2012 | (Family: none) | |
| CN 108887448 A | 27 Nov. 2018 | (Family: none) | |

Form PCT/ISA/210 (patent family annex) (January 2015)

11

**EP 3 881 898 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017186335 A
- JP 2017114888 A
- JP H6141813 A
- WO 2017022030 A